(19) Europäisches Patentamt
European Patent Office
Office européen des brevets

(11) Veröffentlichungsnummer: **0 224 823**
**A2**

(12) **EUROPÄISCHE PATENTANMELDUNG**

(21) Anmeldenummer: **86116185.9**

(22) Anmeldetag: **22.11.86**

(51) Int. Cl.[4]: **C07D 215/48**, A01N 43/42, //C07D417/12,C07D413/04,C07-D401/04,C07D498/04,C07D215-/18

(30) Priorität: **26.11.85 DE 3541722**
**04.03.86 DE 3606949**

(43) Veröffentlichungstag der Anmeldung:
**10.06.87 Patentblatt 87/24**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Hagen, Helmut, Dr.**
**Max-Slevogt-Strasse 17e**
**D-6710 Frankenthal(DE)**
Erfinder: **Eichenauer, Ulrich, Dr.**
**Paul-Ehrlich-Strasse 25a**
**D-6000 Frankfurt 70(DE)**
Erfinder: **Plath, Peter, Dr.**
**Hans-Balcke-Strasse 13**
**D-6710 Frankenthal(DE)**
Erfinder: **Meyer, Norbert, Dr.**
**Dossenheimer Weg 22**
**D-6802 Ladenburg(DE)**
Erfinder: **Wuerzer, Bruno, Dr.**
**Ruedigerstrasse 13**
**D-6701 Otterstadt(DE)**

(54) **Chinolinderivate und ihre Verwendung zur Bekämpfung von unerwümschtem Pflanzenwachstum.**

(57) Chinolinderivate der Formel

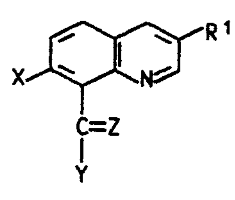

(I),

in der R', X, Y und Z jeweils die in der Beschreibung genannte Bedeutung besitzen, Herbizide, die diese Verbindungen als Wirkstoffe enthalten, sowie ein Verfahren zur Bekämpfung von unerwünschtem Pflanzenwuchs.

EP 0 224 823 A2

**Chinolinderivate und ihre Verwendung zur Bekämpfung von unerwünschtem Pflanzenwachstum**

Die vorliegende Erfindung betrifft neue Chinolinderivate, Herbizide, die diese Verbindungen als Wirkstoffe enthalten, sowie ein Verfahren zur Bekämpfung von unerwünschtem Pflanzenwachstum mittels der neuen Verbindungen.

Es ist bekannt, daß substituierte Chinoline herbizid wirksam sind (DE-A-3 108 873, DE-A-3 210 979, DE-A-3 229 175, DE-A-3 233 089).

Es wurde nun gefunden, daß Chinolinderivate der Formel I

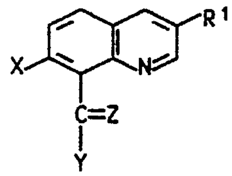

(I),

in der

$R^1$ Halogen oder $C_1$-$C_4$-Alkyl,

X Halogen,

Z Sauerstoff, Hydroxyimino, $C_1$-$C_4$-Alkoxyimino oder einen Hydrazonorest, der durch $C_1$-$C_4$-Alkyl, $C_2$-$C_5$-Alkoxycarbonyl, Arylsulfonyl, 1,3-Oxazol-2-yl oder 1,3-Thiazol-2-yl substituiert ist, wobei die letztgenannten heterocyclischen Reste wiederum durch $C_1$-$C_4$-Alkyl, $C_2$-$C_4$-Hydroxyalkyl oder Hydroxyl substituiert sind, und

Y für den Fall, daß Z die Bedeutung von Sauerstoff besitzt, den Rest $-NR^2R^3$, in dem $R^2$ für Wasserstoff und $R^3$ für $C_1$-$C_4$-Hydroxyalkyl, $C_1$-$C_4$-Alkoxy, $C_2$-$C_4$-Alkenyl, das durch $-C\equiv N$ oder $C_2$-$C_5$-Alkoxycarbonyl substituiert ist, gegebenenfalls substituiertes Arylsulfonyl, 1,3-Oxazol-2-yl oder 1,3-Thiazol-2-yl steht, oder in dem $R^2$ und $R^3$ zusammen für $C_1$-$C_4$-Alkyliden stehen, das durch $C_1$-$C_4$-Dialkylamino, Hydroxyamino oder $C_1$-$C_4$-Alkoxyamino substituiert ist; einen Hydrazinorest, der gegebenenfalls durch $C_2$-$C_5$-Alkylcarbonyl, $C_2$-$C_5$-Alkoxycarbonyl, $C_1$-$C_4$-Acyl, $C_2$-$C_6$-Alkyliden, Thiocarbamoyl, $C_1$-$C_4$-Alkoxy-oxalyl, Amino-oxalyl oder durch gegebenenfalls Methyl-, Chlor-, Brom-oder Nitro-substituiertes Arylsulfonyl substituiert ist; $C_1$-$C_4$-Alkyl, das gegebenenfalls durch $C_2$-$C_5$-Alkoxycarbonyl substituiert ist; oder $C_3$-$C_8$-Cycloalkenyl, das durch $C_1$-$C_4$-Dialkylamino oder $C_4$-$C_6$-Alkylenimino substituiert ist,

für den Fall, daß Z die Bedeutung von $C_1$-$C_4$-Alkoxyimino oder des oben genannten substituierten Hydrazonorests besitzt, Wasserstoff oder Halogen, und

für den Fall, daß Z die Bedeutung von Hydroxyimino besitzt, Halogen, Amino, $C_1$-$C_4$-Alkoxy oder $C_1$-$C_4$-Alkylthio, das durch Carboxyl oder $C_2$-$C_5$-Alkoxycarbonyl substituiert ist, bedeuten,

oder in der die Gruppe

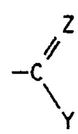

für den Rest $-C\equiv N^{\oplus}-O^{\ominus}$ oder einen fünfgliedrigen Heterocyclus steht, der neben einem Stickstoffatom noch ein oder zwei weitere Heteroatome, ausgewählt aus der Gruppe, bestehend aus Stickstoff, Sauerstoff und Schwefel, sowie eine oder zwei Doppelbindungen aufweist, und der gegebenenfalls substituiert und/oder durch weitere Carbo-oder Heterocyclen anneliert und über ein Kohlenstoffatom an den Chinolinring gebunden ist, herbizid wirksam sind.

$R^1$ in Formel I bedeutet z.B. Fluor, Chlor, Brom, Methyl, Ethyl, Propyl, Isopropyl, Butyl, Isobutyl, But-2-yl oder tert-Butyl, vorzugsweise Chlor; und Methyl.

X in Formel I bedeutet z.B. Fluor, Chlor oder Brom, vorzugsweise Chlor.

Z in Formel I bedeutet z.B. Sauerstoff; Hydroxyimino; Methoxyimino, Ethoxyimino, Propoxyimino oder Butoxyimino; Methylhydrazono, Ethylhydrazono, Isopropylhydrazono, Butylhydrazono, Methoxycarbonylhydrazono, Ethoxycarbonylhydrazono, Propoxycarbonylhydrazono, tert-Butoxycarbonylhydrazono, Phenylsulfonylhydrazono, 2-Methylphenylsulfonylhydrazono, 4-Methylphenylsulfonylhydrazono, 4-Chlorphenylsulfonylhydrazono, 4-Methyl-1,3-thiazol-2-yl-hydrazono, 4-Ethyl-1,3-oxazol-2-yl-hydrazono, 4-tert-Butyl-1,3-thiazol-2-yl-hydrazono, 4-Hydroxy-1,3-oxazol-2-yl-hydrazono, 4-Hydroxyl-1,3-thiazol-2-yl-hydrazono, 4-(2-Hydroxyethyl)-1,3-thiazol-2-yl-hydrazono oder 3-(2-Hydroxyethyl)-4-hydroxy-1,3-thiazol-2-yl-hydrazono.

Die Bedeutung von Y in Formel I richtet sich nach der Bedeutung des Restes Z.

Für den Fall, daß Z die Bedeutung von Sauerstoff besitzt, bedeutet Y z.B. 2-Hydroxyethylamino, (1-Methyl-2-hydroxy)ethylamino, 3-Hydroxypropylamino, (1,1-Dimethyl-2-hydroxy)ethylamino; Methoxyamino, Ethoxyamino, Propoxyamino, Isopropoxyamino, Butoxyamino, Isobutoxyamino; 2-Cyano-2-methylethenylamino, 2-(Methoxycarbonyl)ethenylamino, 2-(Ethoxycarbonyl)-2-methylethenylamino, 3-(Ethoxycarbonyl)prop-2-en-2-ylamino; Phenylsulfonylamino; 2-Methylphenylsulfonylamino, 4-Methylphenylsulfonylamino, 4-Chlorphenylsulfonylamino, 1,3-Oxazol-2-ylamino; 1,3-Thiazol-2-ylamino; Dimethylaminomethylidenamino, 1-(Dimethylamino)ethylidenamino; Hydroxyaminomethylidenamino; 1-(Hydroxyamino)ethylidenamino; Methoxyaminomethylidenamino, 1-(Ethoxyamino)ethylidenamino; Hydrazino; Methoxycarbonylhydrazino, Ethoxycarbonylhydrazino, Isopropoxycarbonylhydrazino, tert-Butoxycarbonylhydrazino; Ethylidenhydrazino, Prop-2-ylidenhydrazino, 2-Methylbut-3-ylidenhydrazino; Thiocarbamoylhydrazino; Methoxyoxalylhydrazino, Ethoxyoxalylhydrazino; Aminooxalylhydrazino; Phenylsulfonylhydrazino, 2-Methylphenylsulfonylhydrazino, 4-Methylphenylsulfonylhydrazino, 4-Chlorphenylsulfonylhydrazino, 4-Bromphenylsulfonylhydrazino, 4-Nitrophenylsulfonylhydrazino; Methyl, Ethyl, Propyl, Butyl; Ethoxycarbonylmethyl; 2-Dimethylaminocyclopent-1-en-1-yl, 2-Diethylaminocyclopent-1-en-1-yl, 2-Pyrrolidinocyclopent-1-en-1-yl, 2-Pyrrolidinocyclohex-1-en-1-yl oder 2-Piperidinocyclohex-1-en-1-yl.

Für den Fall, daß Z die Bedeutung von $C_1$-$C_4$-Alkoxyimino oder des oben genannten Hydrazonorests besitzt, bedeutet Y z.B. Wasserstoff, Chlor oder Brom.

Für den Fall, daß Z die Bedeutung von Hydroxyimino besitzt, bedeutet Y z.B. Chlor, Brom, Amino; Methoxy, Ethoxy, Propoxy, Isopropoxy, Butoxy, Isobutoxy; Carboxymethylthio oder Ethoxycarbonylmethylthio.

Die Gruppe

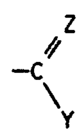

kann auch für den Rest $-C\equiv N^{\oplus}-O^{\ominus}$ stehen oder einen fünfgliedrigen Heterocyclus bedeuten. Dabei kann der Heterocyclus durch $C_1$-$C_4$-Alkyl, das gegebenenfalls durch Hydroxyl oder $C_1$-$C_4$-Alkoxy substituiert ist, $C_1$-$C_4$-Alkoxy, Hydroxyl, Amino, Di-$C_1$-$C_4$-alkyl-amino, $C_4$-$C_6$-Alkylenimino, $C_2$-$C_5$-Alkoxycarbonyl, Carbamoyl oder Cyan substituiert und/oder durch Carbocyclen, wie Cyclopentan oder Cyclohexan, oder durch Heterocyclen, wie Tetrahydrofuran oder Tetrahydropyran, annelliert sein.

Beispielhaft seien folgende heterocyclische Reste für die Gruppe

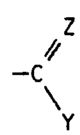

genannt: Triazolyle wie 1,3,4-Triazolyl-2-yl, 1,2,4-Triazol-3-yl, Oxazolyle, wie 1,3-Oxazol-2-yl, 1,2-Oxazol-3-yl, Thiazolyle wie 1,3-Thiazol-2-yl, Oxdiazolyle wie 1,3,4-Oxdiazol-2-yl, partiell hydrierte Oxazolyle wie 4,5-Dihydro-1,3-oxazol-1-yl, 4,5-Dihydro-1,2-oxazol-3-yl, partiell hydrierte Thiazolyle wie 4,5-Dihydro-1,3-thiazol-2-yl, substituierte Triazolyle wie 5-Methyl-1,3,4-triazol-2-yl, substituierte Oxazolyle wie 4,5-Dihydro-4-methyl-1,3-oxazol-2-yl, 4,5-Dihydro-5-ethoxy-1,2-oxazol-3-yl, 5-Methoxymethyl-1,2-oxazol-3-yl, 4-Methoxycarbonyl-5-hydroxy-1,2-oxazol-3-yl, 4-Methoxycarbonyl-5-methyl-1,2-oxazol-3-yl, 4,5-Dihydro-5-hydroxymethyl-1,2-oxazol-3-yl, 4,5-Dihydro-5-methyl-5-methoxycarbonyl-1,2-oxazol-3-yl, 4,5-Dihydro-5-methoxycarbonyl-1,2-oxazol-3-yl, 4,5-Dihydro-4,5-bis(methoxycarbonyl)-1,2-oxazol-3-yl (trans), 4,5-Dihydro-4-cyano-5-amino-1,2-oxazol-3-yl, annellierte Oxazolyle wie 3a,4,5,6-Tetrahydro-6a-pyrrolidino-cyclopenta[d][1,2]oxazol-3-yl, 3a,4,5,6a-Tetrahydro-furo[3,2-d][1,2]oxazol-3-yl, 3a,4,6,6a-Tetrahydrofuro[3,4-d][1,2]oxazol-3-yl, 4H-3a,5,6,7a-Tetrahydro-pyrano[3,2-d][1,2]oxazol-3-yl, substituierte Thiazolyle wie 4,5-Dihydro-4-methyl-1,3-thiazol-2-yl, substituierte Oxdiazolyle wie 5-Ethyl-1,3,4-oxdiazol-2-yl, 5-Hydroxy-1,3,4-oxdiazol-2-yl, 5-Carbamoyl-1,3,4-oxdiazol-2-yl oder 3-Methyl-1,2,4-oxdiazol-5-yl.

Bevorzugt sind Verbindungen in denen $R^1$ und X Halogen, insbesondere Chlor, Z Sauerstoff und Y den Rest $-NHR^3$, wobei $R^3$ für $C_1$-$C_4$-Alkoxy, insbesondere Ethoxy steht, bedeuten.

Die erfindungsgemäßen Carbonsäureamide und Carbonsäurehydrazide (Z = O, Y = $NR^2R^3$ bzw. Hydrazinorest) werden durch Umsetzung des Säurechlorids (Z = O, Y = Cl) mit den betreffenden Aminen bzw. Hydrazinen in einem inerten Lösungsmittel unter Zusatz eines säurebindenden Mittels erhalten.

Als inerte Lösungsmittel kommen chlorierte Kohlenwasserstoffe, wie Methylenchlorid oder Chlorbenzol; Ether, wie Tetrahydrofuran, Dioxan, 1,2-Dimethoxyethan oder Diethylenglykoldimethylether; aromatische Kohlenwasserstoffe, wie Toluol oder Xylol in Frage. Bevorzugt sind 1,2-Dimethoxyethan und Methylenchlorid.

Als säurebindendes Mittel kommen tertiäre Amine, wie Triethylamin, Pyridin oder N,N-Dimethylaminopyridin in Betracht. Zweckmäßig verwendet man 1 bis 2 Mol säurebindendes Mittel pro Mol Säurechlorid.

In manchen Fällen kann auch ein entsprechender Überschuß der Amin-bzw. Hydrazinkomponente vorteilhaft als Säurebinder eingesetzt werden. Bevorzugt verwendet man Triethylamin.

Die Reaktionstemperatur kann innerhalb eines größeren Bereichs variiert werden. Im allgemeinen arbeitet man bei einer Temperatur von -30 bis +80°C, vorzugsweise -10 bis +25°C.

Eine Möglichkeit zur Herstellung von Säureamiden, die das Strukturelement

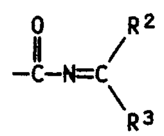

aufweisen ($R^2$, $R^3$ = Alkyliden) besteht darin, das entsprechende freie Amid mit Amidacetalen (z.B. N,N-Dimethylformamid-dimethylacetal oder N,N-Dimethylacetamid-dimethylacetal) oder Orthocarbonsäureestern (z.B. Orthoameisensäuretrimethylester oder Orthoessigsäuretriethylester) umzusetzen.

Als Lösungsmittel kommen aliphatische Amide wie N,N-Dimethylformamid, N,N-Dimethylacetamid sowie aromatische Kohlenwasserstoffe, wie Toluol, Xylol oder Chlorbenzol in Frage. In manchen Fällen ist es auch zweckmäßig, die Umsetzung in überschüssigem Amidacetal oder Orthocarbonsäureester durchzuführen. Der bei der Reaktion entstehende Alkohol kann im Reaktionsgemisch belassen werden oder wird vorzugsweise mittels Destillation entfernt.

Als Reaktionstemperatur wählt man zweckmäßig die Rückflußtemperatur des Reaktionsgemisches, die im allgemeinen bei 100 bis 150°C liegt.

Die erfindungsgemäßen Keto-Verbindungen können in an sich bekannter Weise aus dem Carbonsäureester mittels einer Grignard-Reaktion erhalten werden, wobei es überraschend ist, daß in diesem Fall die Grignard-Reaktion auf der Keto-Stufe stehen bleibt und kein tertiärer Alkohol gebildet wird.

Diejenigen erfindungsgemäßen Verbindungen, die heterocyclische Substituenten tragen, können nach an sich bekannten Methoden der Heterocyclensynthese, wie sie z.B. in R.A. Katritzky, C.W. Rees "Comprehensive Heterocyclic Chemistry", Band 4 und 5, Pergamon Press 1984 und A. Weissberger "Chemistry of Heterocyclic Compounds", Interscience Publishers beschrieben sind, erhalten werden.

Für den Fall, daß die Gruppe

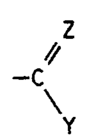

für den Rest $-C{\equiv}N^{\oplus}-O^{\ominus}$ steht, stellt man das Nitriloxid ausgehend vom Oxim über das Hydroxamsäurechlorid als Zwischenstufe und sich anschließender Chlorwasserstoffabspaltung her.

Die weiteren erfindungsgemäßen Verbindungen (z.B. Oxime, Hydrazone oder Oximester) können ebenfalls nach an sich bekannten Methoden, wie sie beispielsweise in Houben-Weyl, "Methoden der organischen Chemie", Band 10/2 und 10/4 beschrieben sind, erhalten werden.

Die Chinolinderivate I weisen eine herbizide Wirkung auf und sind gegenüber Kulturpflanzen selektiv.

Die Chinolinderivate der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-oder Emulgiermittel in Wasser homogenisiert werden. Es können aber auch aus wirksamer Substanz Netz-, Haft-, Dispergier-oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl-oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu-und Stäubemittel können durch Mischen oder gemeinsames Vermahlen der wirksamen Substanzen mit einem festen Trägerstoff hergestellt werden.

Granulate, z.B. Umhüllungs-, Imprägnierungs-und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium-und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Applikation kann im Vorauflauf-oder im Nachauflaufverfahren erfolgen, vorzugsweise im Nachauflaufverfahren. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,1 bis 5,0, vorzugsweise 0,25 bis 3,0 kg/ha.

In Anbetracht des erfaßbaren Wirkungsspektrums zur Unkrautbekämpfung, der Verträglichkeit der Kulturpflanzen oder der erwünschten Beeinflussung des Wachstums derselben sowie angesichts der Vielfalt der Applikationsmethoden können die erfindungsgemäßen Verbindungen je nach Substitutionsmuster in einer großen Zahl von Kulturpflanzen eingesetzt werden.

In Betracht kommen beispielsweise folgende Kulturen:

| Botanischer Name | Deutscher Name |
| --- | --- |
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |

| Botanischer Name | Deutscher Name |
|---|---|
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen Chinolincarbonsäurederivate mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die nachfolgenden Beispiele sollen die Erfindung näher erläutern.

Beispiel 1

25 g (0,5 mol) Hydrazinhydrat und 50 g (0,5 mol) Triethylamin wurden in 500 ml Dimethoxyethan gelöst. In die Lösung wurden unter Eiskühlung 130 g (0,5 mol) 3,7-Dichlorchinolin-8-carbonsäurechlorid langsam eingetragen, so daß die Temperatur 20°C nicht überstieg. Nach 3-stündigem Rühren bei Raumtemperatur wurde das Lösungsmittel am Rotationsverdampfer entfernt, der Rückstand in Wasser aufgenommen und abgesaugt. Man erhielt 101,5 g (79 %) 3,7-Dichlorchinolin-8-carbonsäurehydrazid vom Schmp. 136°C (Verbindung Nr. 1).

Die nachfolgenden Beispiele in Tabelle 1 wurden in analoger Weise erhalten.

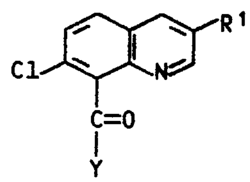

(Ia)

| Beispiel Nr. Verbindung Nr. | $R^1$ | Y | Schmp. [°C] |
|---|---|---|---|
| 2 | Cl | $-NH-NHCOCH_3$ | 287 |
| 3 | $CH_3$ | $-NH-NHCO_2C(CH_3)_3$ | 120 |
| 4 | Cl | $-NH-NHCOCONH_2$ | 205 |
| 5 | Cl | $-NH-NHCOCO_2C_2H_5$ | 183 |
| 6 | Cl | $-NH-NHCSNH_2$ | 230 |
| 7 | Cl | $-NH-NHSO_2C_6H_4CH_3$ (p) | 210 |
| 8 | $CH_3$ | $-NH-NH_2$ | 205 |
| 9 | $CH_3$ | $-NH-NHCOCONH_2$ | 231 |
| 10 | $CH_3$ | $-NH-NHCSNH_2$ | 220 |
| 11 | $CH_3$ | $-NH-NHSO_2C_6H_4CH_3$ (p) | 228 |
| 12 | Cl | $-NH-C(CH_3)_2CH_2OH$ | 215 |
| 13 | Cl | $-NH-CH_2CH(CH_3)OH$ | 100 |
| 14 | Cl | $-NH-CH_2CH_2OH$ | 179 |
| 15 | $CH_3$ | $-NH-C(CH_3)_2CH_2OH$ | 165 |
| 16 | $CH_3$ | $-NH-CH(CH_3)CH_2OH$ | 140 |
| 17 | $CH_3$ | $-NH-CH_2CH_2OH$ | 168 |
| 18 | $CH_3$ | $-NH-SO_2-C_6H_5$ | 220 |
| 19 | Cl | $-NH-SO_2-C_6H_5$ | 163 |
| 20 | Cl | —NH— 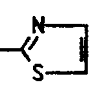 | 260 |

Beispiel 21

3,7-Dichlorchinolin-8-hydroxamsäure-O-ethylester

13,0 g (50 mmol) 3,7-Dichlorchinolin-8-carbonsäurechlorid wurden in 50 ml Dimethoxyethan suspendiert und 5,8 g (60 mmol) O-Ethylhydroxylamin-hydrochlorid sowie 15 g (150 mmol) Triethylamin zugegeben. Nach 5-stündigem Rühren bei Raumtemperatur wurde das Lösungsmittel am Rotationsverdampfer entfernt, der Rückstand mit Wasser verrieben und abgesaugt. Man erhielt 11,0 g (77 %), Schmp.: 140°C (Verbindung Nr. 21).

Beispiel 22

N,N-Dimethyl-N'-(7-Chlor-3-methyl-8-chinolinoyl)-formamidin

Man löste 55,1 g (0,25 mol) 7-Chlor-3-methylchinolin-8-carboxamid in 50 ml N,N-Dimethylformamid, gab 39,3 g (0,32 mol) N,N-Dimethylformamiddimethylacetal zu und erhitzte 4 Stunden unter Rückfluß. Nach Fällen auf Ether isolierte man 44,0 g (64 %) farblose Kristalle, Schmp. 183 bis 185°C (Zers.) (Verbindung Nr. 22)

Die nachfolgenden Beispiele in Tabelle 2 wurden in analoger Weise erhalten.

Tabelle 2

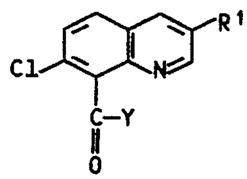

(Ib)

| Beispiel Nr. Verbindung Nr. | $R^1$ | Y | Schmp. [°C] |
|---|---|---|---|
| 23 | $CH_3$ | $-N{=}C(CH_3)-N(CH_3)_2$ | 141 |
| 24 | Cl | $-N{=}C(CH_3)-N(CH_3)_2$ | 205 |
| 25 | Cl | $-N{=}CH-N(CH_3)_2$ | 235 |

Beispiel 26

N-(7-Chlor-3-methyl-8-chinolinoyl)-formamidoxim

13,8 g (50 mmol) Amidin aus Beispiel 22 suspendierte man in 50 ml Eisessig, gab 4,2 g (60 mmol) Hydroxylammoniumhydrochlorid und 5,0 g (60 mmol) Natriumacetat zu, erhitzte 5 Stunden unter Rückfluß, fällte auf Wasser und saugte ab. 6,0 g (45 %), Schmp. 235-238°C (Verbindung Nr. 26).

Die entsprechende 3-Chlorchinolinverbindung wurde analog erhalten, Schmp. 220 bis 230°C (Verbindung Nr. 27).

Beispiel 28

2-(7-Chlor-3-methylchinolin-8-yl)-1,3,4-oxdiazol

12,0 g (50 mmol) 7-Chlor-3-methylchinolin-8-carbonsäurehydrazid (Verbindung Nr. 8) wurden in Triethylorthoformiat 3 Stunden auf 120°C erhitzt und der entstehende Alkohol abdestilliert. Abziehen des überschüssigen Orthoesters und Anreiben mit Ether lieferte 9,4 g (77 %), Schmp. 165°C (Verbindung Nr. 28).

Die Verbindungen Ic der Tabelle 3 wurden analog erhalten.

Tabelle 3

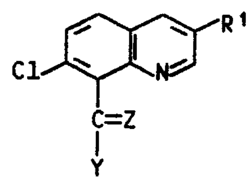

(Ic)

| Beispiel Nr. Verbindung Nr. | $R^1$ | 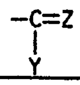 | Schmp. [°C] |
|---|---|---|---|
| 29 | $CH_3$ | 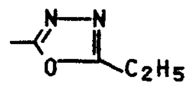 | 150 |
| 30 | Cl | 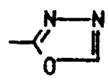 | 218 |
| 31 | Cl | 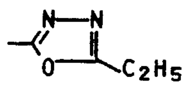 | 131 |

Beispiel 32

5-(3,7-Dichlorchinolin-8-yl)-1,3,4-oxdiazol-2-carboxamid

26,0 g (80 mmol) von Verbindung Nr. 4 rührte man 6 Stunden bei 130°C in 100 g Polyphosphorsäure. Danach rührte man in Wasser ein und ließ über Nacht auskristallisieren. 16,3 g (66 %), Schmp. 220°C - (Zers.) (Verbindung Nr. 31).

Beispiel 33

5-(3,7-Dichlorchinolin-8-yl)-1,3,4-oxdiazol-2-on

12,8 g (50 mmol) 3,7-Dichlorchinolin-8-carbonsäurehydrazid (Verbindung Nr. 1) wurden in 100 ml Dimethoxyethan suspendiert, 7,9 g (100 mmol) Pyridin zugegeben und danach Phosgen über einen Zeitraum von 4 Stunden bei 25°C eingeleitet. Nach Entfernen von überschüssigem Phosgen und Lösungsmittel rührte man in Wasser ein, und saugte ab: 8,7 g (62 %), Schmp.: 87°C (Verbindung Nr. 33).

Analog gewann man das 3-Methylderivat. Schmp.: 232°C (Verbindung Nr. 34).

Beispiel 35

4,5-Dihydro-2-(3,7-dichlorchinolin-8-yl)-oxazol

20,9 g (80 mmol) Verbindung Nr. 14 wurden in 50 ml Thionylchlorid 4 Stunden unter Rückfluß erhitzt. Man fällte auf Eis und gewann 18,3 g (86 %), Schmp.: 145°C (Verbindung Nr. 35).

Analog wurden die in Tabelle 4 aufgeführten Verbindungen Ic hergestellt.

Tabelle 4

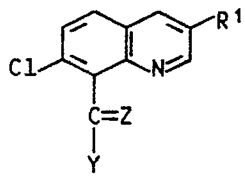

(Ic)

| Beispiel Nr. Verbindung Nr. | $R^1$ | 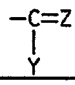 | | Schmp. [°C] |
|---|---|---|---|---|
| 36 | Cl | 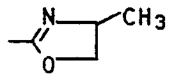 | | 150 |
| 37 | $CH_3$ | 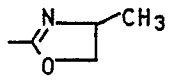 | x HCl | 218 |
| 38 | $CH_3$ | 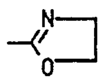 | x HCl | 220 |

Beispiel 39

3-(7-Chlor-3-methylchinolin-8-yl)-1,2,4-triazol

13,8 g (50 mmol) Verbindung Nr. 21 wurden mit 3,0 (60 mmol) Hydrazinhydrat in 50 ml Eisessig 4 Stunden bei 70°C gerührt. Nach Fällen auf Wasser und Absaugen isolierte man 9,5 g (78 %), Schmp. 274-276°C (Verbindung Nr. 39).

In analoger Weise stellte man die Verbindung Ic der Tabelle 5 dar.

Tabelle 5

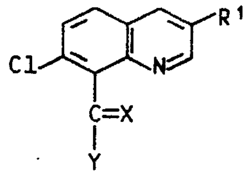

(Ic)

| Beispiel Nr. Verbindung Nr. | $R^1$ | 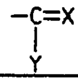 | Schmp. [°C] |
|---|---|---|---|
| 40 | Cl | 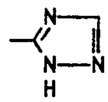 | 280 |
| 41 | Cl | 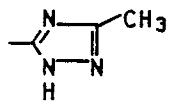 | 218 |
| 42 | Cl | 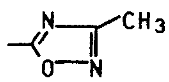 | 125 |
| 43 | $CH_3$ | 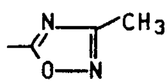 | 145 |
| 44 | $CH_3$ | 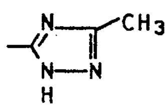 | 238 |

Beispiel 45

3,7-Dichlorchinolin-8-hydroxamsäurechlorid-hydrochlorid

In eine Suspension von 48,2 g (0,2 mol) 3,7-Dichlorchinolin-8-aldoxim in Chloroform leitete man 3 Stunden bei 40°C Chlor ein, saugte ab, wusch mit Ether und trocknete: 59,3 g (95 %), Schmp.: > 300°C - (Verbindung Nr. 45).

Analog wurde erhalten:

3,7-Dichlorchinolin-8-hydroxamsäure-bromid-hydrobromid (Verbindung Nr. 46).

Beispiel 47

3,7-Dichlorchinolin-8-nitriloxid

15,6 g (50 mmol) der Verbindung Nr. 45 wurden in 100 ml 10 %ige Natriumcarbonat-Lösung eingerührt, 2 Stunden bei Raumtemperatur gerührt und abgesaugt: 108 g (90 %), Schmp.: 185°C (Verbindung Nr. 47).

Das Nitriloxid ist in analoger Weise auch aus Verbindung Nr. 46 zugänglich.

Beispiel 48

3-(3',7'-Dichlorchinolin-8'-yl)-1,2-oxazol-4,5-dicarbonsäure-dimethylester

12,0 g (50 mmol) 3,7-Dichlorchinolin-8-nitriloxid (Verbindung Nr. 47) wurden in 100 ml Dimethoxyethan suspendiert, 8,50 g (60 ml) Acetylendicarbonsäuredimethylester zugegeben und 4 Stunden unter Rückfluß erhitzt. Nach Fällen auf Wasser, Absaugen und Trocknen erhielt man 17,8 g (93 %), Schmp.: 135°C - (Verbindung Nr. 48).

In analoger Weise erhielt man die in der Tabelle 6 aufgeführten Verbindungen Id.

Tabelle 6

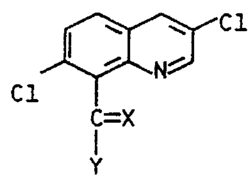

(Id)

| Beispiel Nr. Verbindung Nr. | 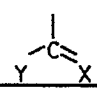 | Schmp. [°C] |
|---|---|---|
| 49 | 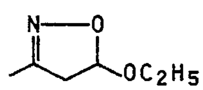 | 145 |

Tabelle 6: Fortsetzung

| Beispiel Nr. Verbindung Nr. | 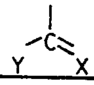 | Schmp. [°C] |
|---|---|---|
| 50 | 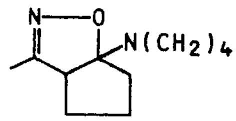 | 105 |
| 51 | 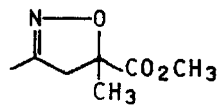 | 229 |
| 52 | 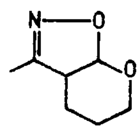 | 135 |
| 53 | 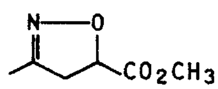 | 135 |
| 54 | 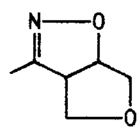 | 125 |
| 55 | 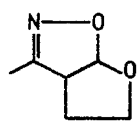 | 164 |
| 56 | 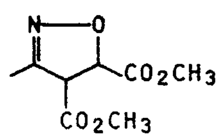 | 78 |
| 57 | 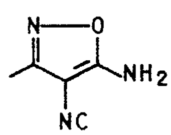 | 200 |
| 58 | 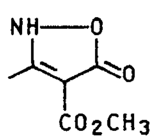 | 180 |

Beispiel 59

3,7-Dichlorchinolin-8-hydroxamsäure-(carboxymethylthio)ester

12,0 g (50 mmol) 3,7-Dichlorchinolin-8-nitriloxid (Verbindung Nr. 47) wurden in 100 ml Dimethoxyethan suspendiert und 5,6 g (60 mmol) Mercaptoessigsäure zugefügt. Nach 6stündigem Rühren bei Raumtemperatur wurde vom Lösungsmittel befreit, mit Wasser versetzt und abgesaugt. 13,5 g (81 %), Schmp.: 174°C - (Verbindung Nr. 59).

Analog wurden die in der Tabelle 7 aufgeführten Verbindungen Ie erhalten.

Tabelle 7

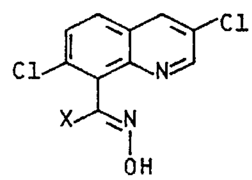

(Ie)

| Beispiel Nr. Verbindung Nr. | X | Schmp. [°C] |
|---|---|---|
| 60 | $NH_2$ | 112 |
| 61 | $OCH_3$ | 130 |

Beispiel 62

3,7-Dichlor-8-propionylchinolin

Die Lösung eines Grignard-Reagenzes, hergestellt aus 7,2 g (60 mmol) Ethylbromid und 1,6 g (60 mmol) Magnesium-Spänen in 30 ml abs. Tetrahydrofuran, wurde bei -70°C zu einer Suspension von 6,4 g - (25 mmol) 3,7-Dichlorchinolin-8-carbonsäuremethylester in 25 ml abs. Tetrahydrofuran getropft, und das Gemisch langsam auf Raumtemperatur gebracht. Danach goß man auf Eis, säuerte mit 10 % Salzsäure an, saugte ab und trocknete: 4,5 g (71 %), Schmp.: 108-110°C (Verbindung Nr. 62).

Analog wurde 7-Chlor-3-methyl-8-propionylchinolin (Verbindung Nr. 63) erhalten.

Beispiel 64

3-(7′-Chlor-3′-methylchinolin-8′-yl)propan-3-oncarbonsäureethylester

70,8 g (0,3 mol) 7-Chlor-3-methylchinolin-8-carbonsäuremethylester wurden in 150 ml Essigester suspendiert, 16 g 80 % Natriumhydrid zugegeben und das Gemisch 5 Stunden unter Rückfluß erhitzt. Nach Fällen auf Eis und Ansäuern mit verd. Salzsäure wurde abgesaugt und getrocknet, 51 g (58 %), Schmp.: 143-145°C (Verbindung Nr. 64).

Anwendungsbeispiele

Die herbizide Wirkung der substituierten Chinolin-8-carbonsäureamide der Formel I auf das Wachstum der Testpflanzen wurde durch folgende Gewächshausversuche gezeigt.

Als Kulturgefäße dienten Plastikblumentöpfe mit 300 cm³ Inhalt und lehmigem Sand mit etwa 3 % Humus als Substrat. Bei Soja wurde etwas Torfmull zugesetzt, um einen besseren Stand zu erzielen. Die Samen der Testpflanzen wurden nach Arten getrennt eingesät.

Bei Vorauflaufbehandlung wurden die Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Die Aufwandmengen betrugen 0,5 und 2,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung zog man die Testpflanzen je nach Wuchsform erst bis zu einer Wuchshöhe von 3 bis 15 cm an und behandelte sie danach. Es wurden direkt gesäte und in den gleichen Gefäßen aufgewachsene Pflanzen ausgewählt, oder sie wurden erst als Keimpflanzen getrennt angezogen. Die Aufwandmengen für die Nachauflaufbehandlung variierten je nach Wirkstoff, sie betrugen 2,0 und 3,0 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 36°C) und für solche gemäßigter Klimate 10 bis 20°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

In den Versuchen verwendete man folgende Pflanzenarten:

| Lateinischer Name | Deutscher Name |
|---|---|
| Avena sativa | Hafer |
| Cassia tora | - |
| Centaurea cyanus | Kornblume |
| Echinochloa crus galli | Hühnerhirse |
| Galium aparine | Klettenlabkraut |
| Glycine max | Sojabohnen |
| Ipomoea spp. | Prunkwindearten |
| Oryza sativa | Reis |
| Veronica spp. | Ehrenpreisarten |
| Zea mays | Mais |

Bei Nachauflaufapplikation von 3,0 kg Wirkstoff/ha übertrafen beispielsweise die Verbindungen 1 und 7 in ihrer herbiziden Wirkung gegen dikotyle unerwünschte Pflanzen den bekannten Wirkstoff A (Beispiel 34 aus EP-PS-60 429) deutlich.

Die Verbindungen 4 und 10 eigneten sich zur Bekämpfung von Galium aparine im Nachauflaufverfahren mit 3,0 kg Wirkstoff/ha ohne der Kulturpflanze Hafer Schäden zuzufügen.

Verbindung 7 wurde im Nachauflaufverfahren zur Bekämpfung von ausgewählten dikotylen und monokotylen Pflanzen eingesetzt. Bei 2,0 kg Wirkstoff/ha wurden weder Soja-noch Reiskulturen nennenswert beeinträchtigt und Echinochloa crus-galli und Cassia tora hervorragend bekämpft.

Im Vorauflaufverfahren zeigte die Verbindung 1 bei 0,5 kg Wirkstoff/ha und die Verbindung 7 bei 2,0 kg Wirkstoff/ha starke herbizide Eigenschaften gegenüber dikotylen, unerwünschten Pflanzen. Die Kulturpflanze Mais wurde nicht geschädigt.

Auch bei Vorauflaufapplikation eignete sich Verbindung 7 zur Bekämpfung von Schadpflanzen, wie Echinochloa crus-galli und Ipomoea, wobei die Sojapflanzen unbeeinflußt blieben.

Die Verbindung 32 eignete sich sowohl bei Vorauflauf-als auch bei Nachauflaufapplikation bei 1,0 kg Wirkstoff/ha gegenüber Echinochloa crus-galli und Veronica spp. unter allenfalls unwesentlicher Beeinträchtigung der Kulturpflanze Weizen.

## Ansprüche

1. Chinolinderivate der Formel I

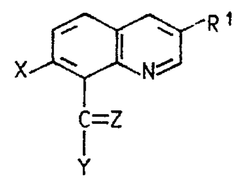

(I),

in der

$R^1$ Halogen oder $C_1$-$C_4$-Alkyl,

X Halogen,

Z Sauerstoff, Hydroxyimino, $C_1$-$C_4$-Alkoxyimino oder einen Hydrazonorest, der durch $C_2$-$C_5$-Alkyl, $C_1$-$C_4$-Alkoxycarbonyl, Arylsulfonyl, 1,3-Oxazol-2-yl oder 1,3-Thiazol-2-yl substituiert ist, wobei die letztgenannten heterocyclischen Reste wiederum durch $C_1$-$C_4$-Alkyl, $C_2$-$C_4$-Hydroxyalkyl oder Hydroxyl substituiert sind, und

Y für den Fall, daß Z die Bedeutung von Sauerstoff besitzt, den Rest $-NR^2R^3$, in dem $R^2$ für Wasserstoff und $R^3$ für $C_1$-$C_4$-Hydroxyalkyl, $C_1$-$C_4$-Alkoxy, $C_2$-$C_4$-Alkenyl, das durch $-C{\equiv}N$ oder $C_2$-$C_5$-Alkoxycarbonyl substituiert ist, gegebenenfalls substituiertes Arylsulfonyl, 1,3-Oxazol-2-yl oder 1,3-Thiazol-2-yl steht, oder in dem $R^2$ und $R^3$ zusammen für $C_1$-$C_4$-Alkyliden stehen, das durch $C_1$-$C_4$-Dialkylamino, Hydroxyamino oder $C_1$-$C_4$-Alkoxyamino substituiert ist; einen Hydrazinorest, der gegebenenfalls durch $C_2$-$C_5$-Alkylcarbonyl, $C_2$-$C_5$-Alkoxycarbonyl, $C_1$-$C_4$-Acyl, $C_2$-$C_6$-Alkyliden, Thiocarbamoyl, $C_1$-$C_4$-Alkoxy-oxalyl, Amino-oxalyl oder gegebenenfalls Methyl-, Chlor-, Brom-oder Nitro-substituiertes Arylsulfonyl substituiert ist; $C_1$-$C_4$-Alkyl, das gegebenenfalls durch $C_2$-$C_5$-Alkoxycarbonyl substituiert ist; oder $C_3$-$C_8$-Cycloalkenyl, das durch $C_1$-$C_4$-Dialkylamino oder $C_4$-$C_6$-Alkylenimino substituiert ist,

für den Fall, daß Z die Bedeutung von $C_1$-$C_4$-Alkoxyimino oder des oben genannten substituierten Hydrazonorests besitzt, Wasserstoff oder Halogen, und

für den Fall, daß Z die Bedeutung von Hydroxyimino besitzt, Halogen, Amino, $C_1$-$C_4$-Alkoxy oder $C_1$-$C_4$-Alkylthio, das durch Carboxyl oder $C_2$-$C_5$-Alkoxycarbony substituiert ist, bedeuten,

oder in der die Gruppe

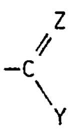

für den Rest $-C{\equiv}N^{\oplus}-O^{\ominus}$ oder einen fünfgliedrigen Heterocyclus steht, der neben einem Stickstoffatom noch ein oder zwei weitere Heteroatome, ausgewählt aus der Gruppe, bestehend aus Stickstoff, Sauerstoff und Schwefel, sowie eine oder zwei Doppelbindungen aufweist, und der gegebenenfalls substituiert und/oder durch weitere Carbo-oder Heterocyclen anneliert und über ein Kohlenstoffatom an den Chinolinring gebunden ist.

2. Chinolinderivate der Formel I nach Anspruch 1, in der

$R^1$ Chlor oder Methyl,

X Chlor,

Z Sauerstoff, Hydroxyimino, $C_1$-$C_4$-Alkoxyimino oder einen Hydrazonorest, der durch $C_2$-$C_5$-Alkyl, $C_1$-$C_4$-Alkoxycarbonyl, Arylsulfonyl, 1,3-Oxazol-2-yl oder 1,3-Thiazol-2-yl substituiert ist, wobei die letztgenannten heterocyclischen Reste wiederum durch $C_1$-$C_4$-Alkyl, $C_2$-$C_4$-Hydroxyalkyl oder Hydroxyl substituiert sind, und

Y für den Fall, daß Z die Bedeutung von Sauerstoff besitzt, den Rest $-NR^2R^3$, in dem $R^2$ für Wasserstoff und $R^3$ für $C_1$-$C_4$-Hydroxyalkyl, $C_1$-$C_4$-Alkoxy, $C_2$-$C_4$-Alkenyl, das durch $-C{\equiv}N$ oder $C_2$-$C_5$-Alkoxycarbonyl substituiert ist, gegebenenfalls substituiertes Arylsulfonyl, 1,3-Oxazol-2-yl oder 1,3-Thiazol-2-yl steht, oder in dem $R^2$ und $R^3$ zusammen für $C_1$-$C_4$-Alkyliden stehen, das durch $C_1$-$C_4$-Dialkylamino, Hydroxyamino oder $C_1$-$C_4$-Alkoxyamino substituiert ist; einen Hydrazinorest, der gegebenenfalls durch $C_2$-$C_5$-Alkylcarbonyl, $C_2$-$C_5$-Alkoxycarbonyl, $C_1$-$C_4$-Acyl, $C_2$-$C_6$-Alkyliden, Thiocarbamoyl, $C_1$-$C_4$-Alkoxy-oxalyl, Amino-oxalyl oder gegebenenfalls Methyl-, Chlor-, Brom-oder Nitro-substituiertes Arylsulfonyl substituiert ist; $C_1$-$C_4$-Alkyl, das gegebenenfalls durch $C_2$-$C_5$-Alkoxycarbonyl substituiert ist; oder $C_3$-$C_8$-Cycloalkenyl, das durch $C_1$-$C_4$-Dialkylamino oder $C_4$-$C_6$-Alkylenimino substituiert ist,

für den Fall, daß Z die Bedeutung von $C_1$-$C_4$-Alkoxyimino oder des oben genannten substituierten Hydrazonorests besitzt, Wasserstoff oder Halogen, und

für den Fall, daß Z die Bedeutung von Hydroxyimino besitzt, Halogen, Amino, $C_1$-$C_4$-Alkoxy oder $C_1$-$C_4$-

Alkylthio, das durch Carboxyl oder $C_2$-$C_5$-Alkoxycarbonyl substituiert ist, bedeuten, oder in der die Gruppe

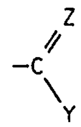

für den Rest -C≡N$^{\oplus}$-O$^{\ominus}$ oder einen fünfgliedrigen Heterocyclus steht, der neben einem Stickstoffatom noch ein oder zwei weitere Heteroatome, ausgewählt aus der Gruppe, bestehend aus Stickstoff, Sauerstoff und Schwefel, sowie eine oder zwei Doppelbindungen aufweist, und der gegebenenfalls substituiert und/oder durch weitere Carbo-oder Heterocyclen anneliert und über ein Kohlenstoffatom an den Chinolinring gebunden ist.

3. Chinolinderivate der Formel I nach Anspruch 1, in der
$R^1$ Halogen
X Halogen
Z Sauerstoff
Y den Rest $-NHR^3$ und
$R^3$ $C_1$-$C_4$-Alkoxy bedeutet.

4. Chinolinderivate der Formel I nach Anspruch 1, in der
$R^1$ Chlor
X Chlor
Z Sauerstoff
Y den Rest $-NHR^3$ und
$R^3$ Ethoxy bedeutet.

5. Herbizid, enthaltend ein Chinolinderivat der Formel I gemäß Anspruch 1.

6. Herbizid, enthaltend inerte Zusatzstoffe und ein Chinolinderivat der Formel I gemäß Anspruch 1.

7. Verfahren zur Bekämpfung von unerwünschtem Pflanzenwachstum, dadurch gekennzeichnet, daß man die unerwünschten Pflanzen und/oder die von unerwünschtem Pflanzenwachstum freizuhaltende Fläche mit einer herbizid wirksamen Menge eines Chinolinderivats der Formel I gemäß Anspruch 1 behandelt.